Europäisches Patentamt

European Patent Office

Office européen des brevets

(11) Veröffentlichungsnummer : **0 057 266**
**B1**

(12) **EUROPÄISCHE PATENTSCHRIFT**

(45) Veröffentlichungstag der Patentschrift :
**09.01.85**

(21) Anmeldenummer : **81109057.0**

(22) Anmeldetag : **28.10.81**

(51) Int. Cl.⁴ : **A 61 K 7/00, A 61 K 7/48,
A 61 K 7/50, A 61 K 7/035**

(54) **Kosmetische Mine für einen Puderstift oder dergleichen.**

(30) Priorität : 30.01.81 DE 3103128

(43) Veröffentlichungstag der Anmeldung :
**11.08.82 Patentblatt 82/32**

(45) Bekanntmachung des Hinweises auf die Patenterteilung : **09.01.85 Patentblatt 85/02**

(84) Benannte Vertragsstaaten :
**AT BE CH FR GB IT LI NL**

(56) Entgegenhaltungen :
**DE-A- 2 718 158
DE-B- 2 540 877
GB-A- 1 092 726
US-A- 3 800 034
US-A- 4 218 852**

(73) Patentinhaber : **Schwan-STABILO Schwanhäusser
GmbH & Co.
Maxfeldstrasse 3
D-8500 Nürnberg (DE)**

(72) Erfinder : **Hofmann, Hans Joachim, Dr.
Pirckheimer Strasse 42 b
D-8500 Nürnberg (DE)**
Erfinder : **Brüchert, Werner
Blücher Strasse 42
D-8500 Nürnberg (DE)**
Erfinder : **Hempel, Matthias, Dr.
Grossgeschaidt 118
D-8501 Heroldsberg (DE)**
Erfinder : **Weber, Klaus
Hessenring 4
D-6054 Rottgau 1 (DE)**

(74) Vertreter : **LOUIS, PÖHLAU, LOHRENTZ & SEGETH
Ferdinand-Maria-Strasse 6
D-8130 Starnberg (DE)**

**Beschreibung**

Die Erfindung betrifft eine kosmetische Mine (für einen Puderstift od. dgl.), bestehend aus zu einem formhaltigen Körper gebundenen pulverförmigen Bestandteilen, die ein teilchenförmiges Material zur Verhinderung eines Zusetzens der Minenoberfläche durch Fett und Feuchtigkeit (Glazing) beim unmittelbaren Anstreichen der Minenoberfläche an die Haut umfassen und ggf. Zusätze von Bindemitteln, Gleitmitteln oder Haftvermittlern enthalten.

Puderstiftminen der vorstehend angegebenen Art weisen einerseits eine solche Formhaltigkeit und Festigkeit auf, daß sie in Containern, Hülsen od. dgl. lose aufgenommen, aber auch in spitzbaren Kunststoff- oder Holzumhüllungen nach Art von Bleistiften eingeleimt werden und in solcher Form gehandhabt werden können. Andererseits ist ihre Konsistenz so beschaffen, daß sich das Pudermaterial bei der Applikation durch verhältnismässig geringen Druck auf die Haut gleichförmig auftragen lässt. Bekannte Puderstiftminen dieser Art (vgl. z. B. DE-B-2 540 877) bestehen aus pulverförmigen Grundbestandteilen wie Stärke, Talkum, Kaolin, Kalziumkarbonat, Glimmerpulver od. dgl., denen die jeweils erwünschten ebenfalls pulverförmigen Farbpigmente zugesetzt sind. Je nach der Art der Grundbestandteile können die Puderstiftminen auch noch weitere, nicht unbedingt pulverförmige Bestandteile enthalten, zu denen Bindemittel, Gleitmittel oder Haftvermittler zählen.

Kosmetische Minen dieser Art haben den Vorteil, daß der Puder ohne zusätzlichen Applikator unmittelbar von der Minenoberfläche auf die Haut durch Anstreichen aufgetragen werden kann. Jedoch müssen besondere Maßnahmen dagegen getroffen werden, daß sich die Minenoberfläche schon nach kurzer Zeit mit Feuchtigkeit und Fett, die auf der Haut stets vorhanden sind, anreichert. Denn dadurch wird die Minenoberfläche geglättet und verfestigt sich (sog. Glazing), so daß sie schon nach verhältnismässig kurzem Gebrauch kein Pudermaterial mehr auf die Haut abgibt, so daß unter Inkaufnahme eines Materialverlustes eine neue Minenoberfläche durch Anspitzen geschaffen werden muß. Bei einem bekannten Kosmetikstift wird zur Verhinderung des Glazings vorgeschlagen, eine hohe Konzentration an Kreide (Kalziumkarbonat) in der Grössenordnung von 40 bis 90 % in der Minenmasse vorzusehen (US-A 38 00 034). Damit wird beabsichtigt, die auf der Haut vorhandene Feuchtigkeit durch die Kreide zu absorbieren. Dieser Vorschlag hat jedoch keine praktische Bedeutung, weil die Verwendung von Kreide (Kalziumkarbonat) in kosmetischen Pudern physiologische Nachteile hat und deshalb davor gewarnt wird (vgl. Rothemann « Das grosse Rezeptbuch der Haut- und Körperpflegemittel » 4. Aufl., Heidelberg 1969, S. 669). Kreide ist daher, wenn überhaupt, allenfalls in einem Prozentsatz von 10 bis 20 % in der Minenmasse zulässig. In dieser Menge ist jedoch der Adsorptionsgrad der Kreide nicht ausreichend, um das Glazing wirksam zu verhindern. Hinzu kommt, daß Kreide selbst ein ausgesprochenes Weißpigment ist, dessen Verwendung in der angegebenen Konzentration nur sehr helle zarte Pastelltöne ermöglicht und jeden Perlglanz abstumpft, so daß die Farbskala der Puderstifte dadurch eng begrenzt ist.

Der Erfindung liegt daher die Aufgabe zugrunde, eine kosmetische Mine der beschriebenen Art zu schaffen, bei der ohne eine Beeinträchtigung der ansonsten gewünschten Eigenschaften der Mine und der Breite der möglichen Farbskala ein Glazing der Minenoberfläche verhindert werden kann.

Erfindungsgemäß wird diese Aufgabe dadurch gelöst, daß das teilchenförmige Material aus vom Siliciumdioxid abgeleiteten Stoffen, aus Aluminiumoxiden, aus Metallkarbiden, aus Metallen oder aus Mischungen daraus gebildet ist.

Es hat sich überraschenderweise gezeigt, daß durch den Zusatz von derartigen Materialien, die im Vergleich zu den pulverförmigen Grundbestandteilen der Mine eine grössere Härte aufweisen, das Glazing stark herabgesetzt, ja sogar vollständig beseitigt werden kann. Dabei bildet das teilchenförmige Material einen Bestandteil der Mine, der insoweit als neutral zu bezeichnen ist, als er für die Herstellbarkeit, die Haftfähigkeit des Puders, die Bindefähigkeit und dgl. keine Rolle spielt, sondern allein das Verschmieren und Glätten der Minenoberfläche verhindern soll. Das schließt jedoch nicht aus, daß das teilchenförmige Material zugleich auch weitere Funktionen erfüllen kann.

Nach einer vorteilhaften Weiterbildung der Erfindung sollte das teilchenförmige Material eine Härte von mindestens 4,0 gemessen nach der MOHS-Härteskala aufweisen, wobei die Härte auf die Teilchen selbst bezogen ist. Das teilchenförmige Material kann nämlich auch durch Mahlen eines porösen oder schwammartig verfestigten Grundmaterials, z. B. Bims, erhalten werden, das in seiner massiven Form aufgrund seiner porösen Struktur eine geringere MOHS-Härte aufweist, wohingegen die das Grundmaterial aufbauenden Teilchen an sich härter sind. Die oben angegebenen Materialien erfüllen diese Forderungen. Auch Aluminiumoxide und Metallkarbide oder Mischungen davon sind geeignet, wobei unter den Aluminiumoxiden Korund in feinster Verteilung in der Mine eine bevorzugte Stellung einnimmt. Alle diese Stoffe sind in einer Vielfalt von Korngrössen und Korngrössenverteilungen erhältlich. Auch metallische Pulver sind als teilchenförmiges Material grundsätzlich geeignet. So kann z. B. an Pulver aus Aluminium oder Aluminiumlegierungen gedacht werden, bei denen die gewünschte Härte durch eine entsprechende Härtebehandlung des Ausgangsmaterials erzeugt ist.

Da das teilchenförmige Material in feinverteilter Form in der Mine vorliegt, wobei zweckmässigerweise eine Teilchengrösse zwischen 10 und 100 μ eingehalten wird, beeinträchtigt es die von einer solchen Mine üblicherweise erwarteten Eigenschaften nicht. Vorzugsweise wird für das teilchenförmige Material eine Teilchengrösse zwischen 10 und 50 μ gewählt. Es hat sich gezeigt, daß zwischen der Teilchengrösse und deren Härte eine gewisse Korrelation besteht in dem Sinn, daß mit zunehmender Teilchengrösse der von der Erfindung beabsichtigte Erfolg auch schon bei geringerer Härte erzielbar ist.

Es ist weiterhin von Vorteil, wenn die Teilchen des teilchenförmigen Materials eine unregelmässig geformte, vorzugsweise zerklüftete Oberflächengestalt haben. So können die Teilchen beispielsweise eine splitterige Form aufweisen, oder — wenn sie Granulatform haben — eine deutlich ausgeprägte rauhe Oberfläche besitzen. Auch diesbezüglich hat sich herausgestellt, daß der von der Erfindung angestrebte Effekt sich umso deutlicher einstellt, je zerklüfteter oder rauher die Oberflächengestalt der Teilchen ist. Hieraus ergibt sich wiederum eine gewisse Korrelation mit der Härte und der Teilchengrösse in dem Sinn, daß Härte und/oder Teilchengrösse geringer gewählt werden können, je weitgehender der Forderung nach einer zerklüfteten Oberflächengestalt der Teilchen entsprochen ist.

Die Menge, in der das teilchenförmige Material in der Mine enthalten ist, ist nicht ausgesprochen kritisch. Es hat sich jedoch gezeigt, daß je nach Härte, Teilchengrösse und Oberflächengestalt der Teilchen ein Anteil von 20 bis 40 Gew.% ausreichend ist. Da die erfindungsgemäss verwendeten Materialien bezüglich der Farbgebung weitgehend neutral sind, muß bei dieser Zusatzmenge keine Beschränkung bei der Farbwahl in Kauf genommen werden.

Es hat sich gezeigt, daß der von der Erfindung beabsichtigte Erfolg insbesondere bei deren Anwendung auf eine gemäß der DE-PS 25 40 877 zusammengesetzte kosmetische Mine erreicht wird. Eine solche Mine weist als Grundbestandteil vorwiegend Glimmerpulver von einer bestimmten Korngrösse auf oder besteht zumindest aus einem erheblichen Teil dieses Pulvers. Als Bindemittel kommen vorwiegend wasserlösliche Celluloseabkömmlinge wie Methylcellulose, Carboxymethylcellulose (CMC), wasserlösliche Schellackseife, Tragant, Gummiarabicum, Dextrin und andere wasserlösliche Bindemittel in Betracht. Auch Guargum, ein natürliches Hydrocolloid, sowie dessen Derivate sind brauchbar.

Der Einbau des teilchenförmigen Materials in den Grundwerkstoff für die Herstellung der kosmetischen Mine nach der Erfindung erfordert keine besonderen Verfahrensmaßnahmen. Am zweckmässigsten werden alle pulvrigen Bestandteile einschließlich dem nach der Erfindung zuzusetzenden teilchenförmigen Material gut miteinander vermengt, so daß eine homogene Verteilung aller Bestandteile ineinander erzielt wird. Daraufhin wird das so erhaltene Pulvergemisch mit einem wasserhaltigen Bindemittel versetzt und die daraus resultierende kenetbare Masse wiederum zum Zweck der Homogenisierung geknetet. Die Formung zur Mine erfolgt vorzugsweise durch Extrudieren der Masse ; daran schließt sich ein Trockenvorgang an, durch den Wasser entzogen und auf diese Weise der formbeständige Körper der Mine geschaffen wird. Die jeweils zugesetzten Mengen an Wasser sind abhängig von der Art des verwendeten wasserlöslichen Bindemittels und für den Fachmann bekannt, so daß sich eine ins einzelne gehende Erläuterung an dieser Stelle erübrigt.

In der nachfolgenden Tabelle sind vier Beispiele für bevorzugte Zusammensetzungen einer kosmetischen Mine nach der Erfindung angegeben. Die Beispiele sind mit den Nummern 1 bis 4 in den Spalten der Tabelle aufgeführt. Es sind sowohl die prozentualen Zusammensetzungen (Gew.%) als auch die jeweils zugegebenen Teile der einzelnen Bestanteile erwähnt. Die Teilchengrösse der jeweils verwendeten teilchenförmigen Materials liegt in der Hauptsache bei 30 bis 40 μ. Mit allen vier Zusammensetzungen hat sich gezeigt, daß das unerwünschte Verschmieren und Zusetzen der Minenoberfläche vermieden werden kann.

## Tabelle

| Bestandteile | % | Teile Nr. 1 | % | Teile Nr. 2 | % | Teile Nr. 3 | % | Teile Nr. 4 |
|---|---|---|---|---|---|---|---|---|
| Basismaterial (Füllstoffe) : | | | | | | | | |
| Talkum | 22 | 60,0 | 25 | 60,0 | 30 | 50,0 | 20 | 45,0 |
| Kaolin | | | | | 6 | 10,0 | | |
| Glimmer | 30 | 80,0 | 35 | 80,0 | | | | |
| teilchenförmiges Material : | | | | | | | | |
| Bimspuder | 20 | 60,0 | | | | | | |
| Quarzmehl | | | 20 | 50,0 | 30 | 50,0 | | |
| Alu.-Oxid | | | | | | | 30 | 60,0 |
| Gleitmittel : | | | | | | | | |
| Zn-Stearat | | | | | 8 | 11,0 | | |
| Mg-Stearat | 4 | 10,0 | | | | | | |
| Ca-Stearat | | | | | | | 6 | 15,0 |

Tabelle (Fortsetzung)

| Bestandteile | % | Teile Nr. 1 | % | Teile Nr. 2 | % | Teile Nr. 3 | % | Teile Nr. 4 |
|---|---|---|---|---|---|---|---|---|
| Haftvermittler : | | | | | | | | |
| Bentonit | 1 | 1,0 | 1 | 1,0 | | | | |
| Mg-Myristat | 4 | 10,0 | 4 | 10,0 | | | | |
| Mg-Al-Silikagel | | | | | 6 | 10,0 | | |
| Bindemittel : | | | | | | | | |
| CMC | | | | | | | 2 | 4,0 |
| Guargum | 2 | 5,0 | | | | | | |
| PVP hochvisk. | | | | | 3 | 5,0 | | |
| Pigmente : | | | | | | | | |
| Farbpigmente | 12 | 30,0 | 5 | 10,0 | 14 | 20,0 | | |
| Pearlglanzpigm. | | | 10 | 30,0 | | | 42 | 90,0 |
| Weißpigmente | 5 | 10,0 | | | 3 | 4,0 | | |
| Prozente | 100 | | 100 | | 100 | | 100 | |

Die beiliegenden Zeichnungen bzw. Darstellungen dienen der weiteren Erläuterung der Erfindung. Darin zeigen :

Figuren 1a, 1b schematische Darstellungen zweier bekannter Ausführungen von Puderstiften im Längsschnitt mit einer Mine, die erfindungsgemäß aufgebaut ist, und

Figuren 2, 3 stark vergrösserte Mikroskopaufnahmen von nach der Erfindung verwendbarem teilchenförmigem Material, nämlich von Bimspuder bzw. Quarzmehl.

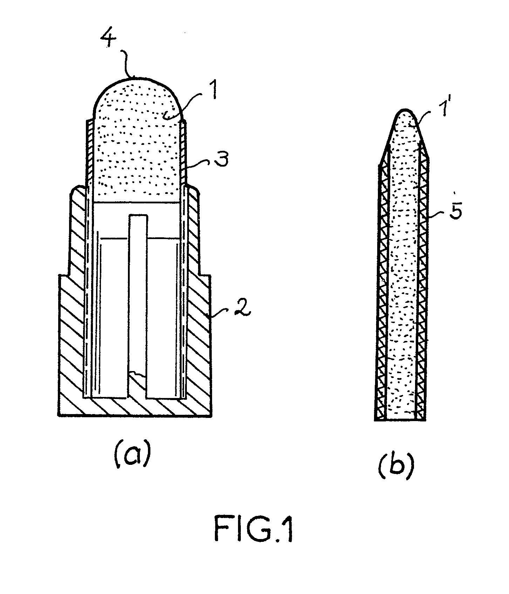

Die Fig. 1a zeigt einen Puderstift, der im wesentlichen aus einer Mine 1 und einem aus Metall oder Kunststoff bestehenden Behälter 2 besteht, in dem eine Schraubhülse 3 aufgenommen ist. Durch Relativverdrehung der Schraubhülse 3 zu dem Behälter 2 lässt sich die Mine 1 in bekannter Weise herausschieben, damit die vordere Oberfläche 4 auf die Haut aufgesetzt und Puder davon aufgetragen werden kann.

Die Fig. 1b zeigt einen Puderstift, dessen Mine 1' in einem aus Holz oder spitzbarem Kunststoff bestehenden Schaft 5 aufgenommen ist, so daß nach dem Verbrauch der Minenspitze durch Anschärfen wieder eine neue Spitze freigelegt werden kann.

Die äußere Erscheinung der Puderstifte nach den Fig. 1a, b unterscheidet sich nicht von derjenigen bekannter Kosmetikstifte.

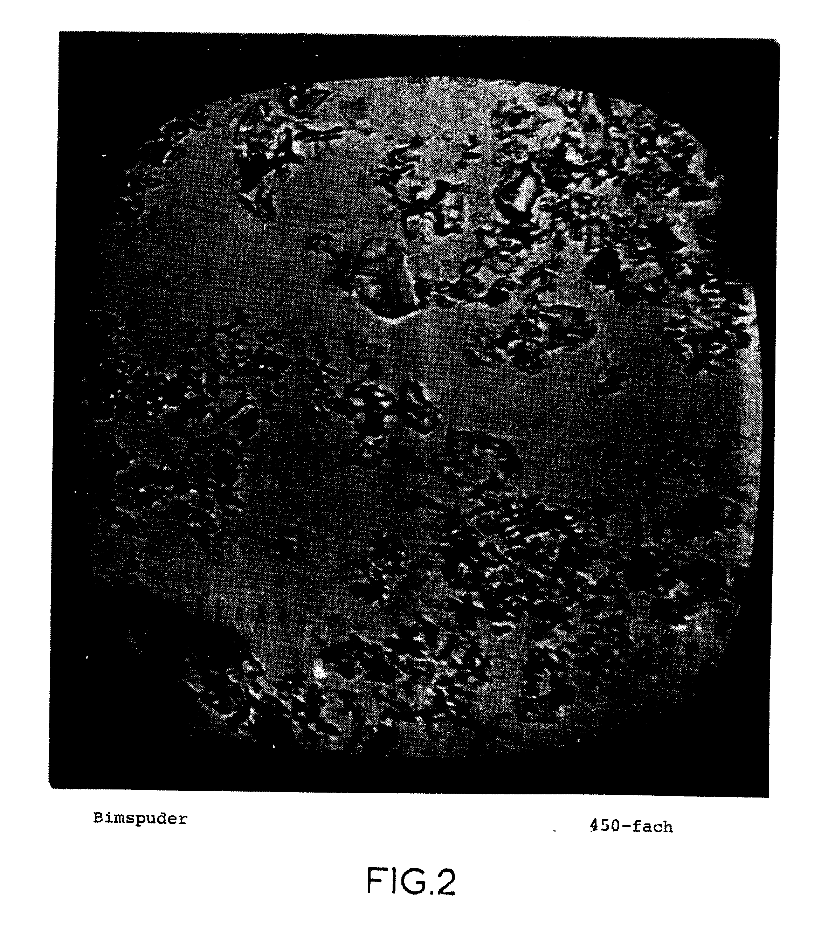

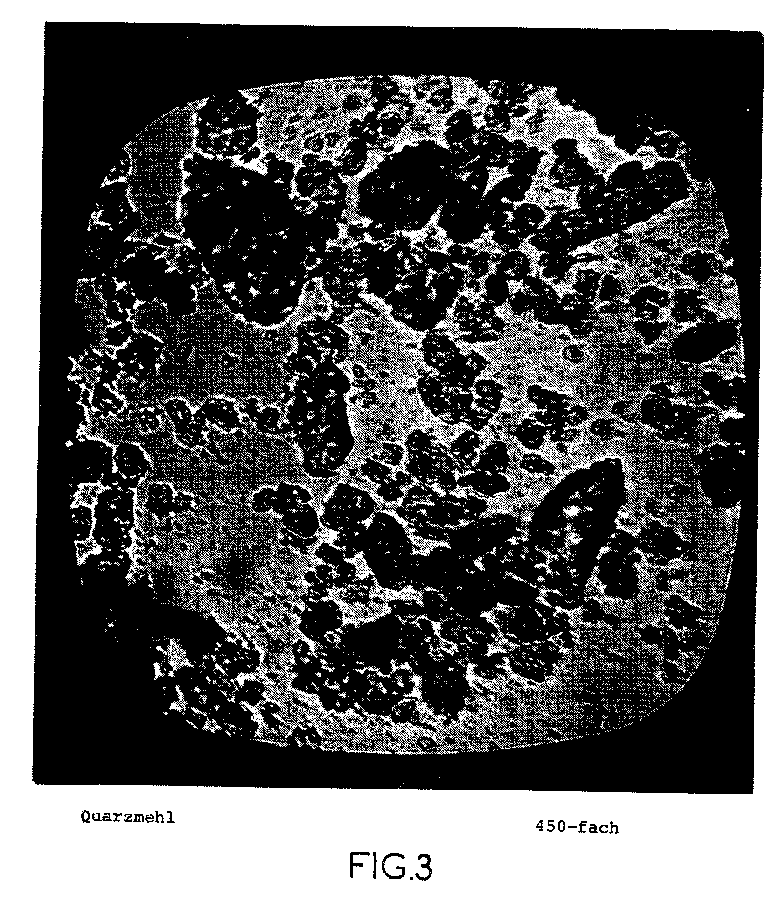

Die Fig. 2 und 3 lassen in 450-facher Vergrösserung die Art des erfindungsgemäß zugesetzten teilchenförmigen Materials erkennen. Der in Fig. 2 dargestellte Bimspuder zeigt deutlich eine splitterige Form der Bimsteilchen mit sehr unregelmässiger zerklüfteter Oberfläche. Die durchschnittliche Grösse der Bimsteilchen liegt bei etwa 30 bis 50 μ. Das aus Fig. 3 ersichtliche Quarzmehl lässt die Granulatkörper-Form der Teilchen erkennen. Deutlich ist auch die Buckligkeit oder Rauhigkeit der Granulatkörper-Oberfläche ersichtlich. Die durchschnittliche Grösse der Teilchen beträgt hier etwa 40 bis 60 μ.

**Ansprüche**

1. Kosmetische Mine, bestehend aus zu einem formhaltigen Körper gebundenen pulverförmigen Bestandteilen, die ein teilchenförmiges Material zur Vermeidung eines Zusetzens der Minenoberfläche durch Fett und Feuchtigkeit (Glazing) beim unmittelbaren Anstreichen der Minenoberfläche an die Haut umfassen und ggf. Zusätze von Bindemitteln, Gleitmitteln oder Haftvermittlern enthalten, dadurch gekennzeichnet, daß das teilchenförmige Material aus vom Siliciumdioxid abgeleiteten Stoffen, aus Aluminiumoxiden, aus Metallkarbiden, aus Metallen oder aus Mischungen daraus gebildet ist.

2. Mine nach Anspruch 1, dadurch gekennzeichnet, daß das teilchenförmige Material aus Quarzmehl, Aluminiumsilicat, Bimspuder oder aus Mischungen daraus besteht.

3. Mine nach Anspruch 1, dadurch gekennzeichnet, daß das teilchenförmige Material aus Korund besteht.

4. Mine nach Anspruch 1 oder 2, dadurch gekennzeichnet, daß die Teilchen des teilchenförmigen Materials eine Härte von mindestens 4,0 nach der MOHS-Härteskala aufweisen.

5. Mine nach einem der Ansprüche 1 bis 4, dadurch gekennzeichnet, daß die Teilchen eine Teilchengrösse zwischen 10 und 50 μ haben.

6. Mine nach einem der Ansprüche 1 bis 5, dadurch gekennzeichnet, daß die Teilchen eine unregelmässig geformte, vorzugsweise zerklüftete Oberflächengestalt haben.

7. Mine nach Anspruch 6, dadurch gekennzeichnet, daß die Teilchen eine splitterige Form haben.

8. Mine nach Anspruch 6, dadurch gekennzeichnet, daß die Teilchen Granulatform mit einer rauhen Oberfläche haben.

9. Mine nach einem der Ansprüche 1 bis 8, dadurch gekennzeichnet, daß das teilchenförmige Material in einem Anteil von 20 bis 40 Gew.% enthalten ist.

## Claims

1. Cosmetic stick core, consisting of pulverulent constituents bonded to form a dimensionally stable body, the constituents containing a particulate material to avoid clogging of the core surface by grease and moisture (glazing) when the core surface directly brushes over the skin, optionally together with the addition of binders, lubricants or adhesion promoters, characterised in that the particulate material is formed of substances derived from silicon dioxide, of aluminium oxides, of metal carbides, of metals or of mixtures of these.

2. Stick core according to Claim 1, characterised in that the particulate material consists of quartz powder, aluminium silicate, pumice powders or mixtures of these.

3. Stick core according to Claim 1, characterised in that the particulate material consists of corundum.

4. Stick core according to Claim 1 or 2, characterised in that the particles of the particulate material have a hardness of at least 4.0 on the MOHS hardness scale.

5. Stick core according to one of Claims 1 to 4, characterised in that the particles have a particle size of between 10 and 50 μ.

6. Stick core according to one of Claims 1 to 5, characterised in that the particles have an irregularly shaped, preferably fissured, surface form.

7. Stick core according to Claim 6, characterised in that the particles have a chipped shape.

8. Stick core according to Claim 6, characterised in that the particles are in the shape of granules having a rough surface.

9. Stick core according to one of Claims 1 to 8, characterised in that the particulate material is present in a proportion of 20 to 40 % by weight.

## Revendications

1. Mine cosmétique composée de constituants pulvérulents liés en un corps de forme stable, qui comportent un matériau particulaire pour éviter une obturation de la surface de la mine par de graisse ou d'humidité (Glazing) lors du frottement de la surface de la mine sur la peau, et éventuellement des additifs liants, des agents lubrifiants ou des agents de cohésion, caractérisée par le fait que le matériau particulaire est constitué par des produits dérivés de silice, par des oxydes d'aluminium, par des carbures métalliques, par des métaux, ou par des mélanges de ces matériaux.

2. Mine selon la revendication 1, caractérisée par le fait que le matériau particulaire est constitué par de la farine de quartz, par du silicate d'aluminium, par de la poudre de pierre ponce, ou par des mélanges de ces matériaux.

3. Mine selon la revendication 1, caractérisée par le fait que le matériau particulaire est constitué de corindon.

4. Mine selon la revendication 1 ou 2, caractérisée par le fait que les particules du matériau particulaire ont une dureté d'au moins 4,0 d'après l'échelle de MOHS.

5. Mine selon l'une quelconque des revendications 1 à 4, caractérisée par le fait que les particules ont une dimension de particules entre 10 et 50 micromètres.

6. Mine selon l'une quelconque des revendications 1 à 5, caractérisée par le fait que les particules ont une forme superficielle irrégulière, de préférence avec une surface crevassée.

7. Mine selon la revendication 6, caractérisée par le fait que les particules ont une forme esquilleuse.

8. Mine selon la revendication 6, caractérisée par le fait que les particules ont une forme granulaire avec une surface grossière.

9. Mine selon l'une quelconque des revendications 1 à 8, caractérisée par le fait que le matériau particulaire est présent à raison de 20 à 40 % en poids.

(a)

(b)

FIG.1

Bimspuder                                            450-fach

# FIG.2

Quarzmehl　　　　　　　　　　　　　450-fach

FIG.3